# EUROPEAN PATENT APPLICATION

(11) **EP 4 480 385 A1**
(43) Date of publication of application: **25.12.2024**
(21) Application number: 23180082.2
(22) Date of filing: 19.06.2023
(51) Int. Cl.: A61B 1/04, A61B 1/06, G01N 21/64, A61B 1/00, G01N 21/00, G02B 21/16

(54) **DATA PROCESSING DEVICE FOR A MEDICAL OBSERVATION DEVICE SUCH AS A MICROSCOPE OR AN ENDOSCOPE AND COMPUTER-IMPLEMENTED METHOD FOR GENERATING A DIGITAL REFLECTANCE COLOR OUTPUT IMAGE**

(71) Applicant: Leica Instruments (Singapore) Pte Ltd, Singapore 608924 (SG)
(72) Inventor: Themelis, George, 608924 Singapore (SG); Pentarakis, Kyriakos, 608924 Singapore (SG)
(74) Representative: Grünecker Patent- und Rechtsanwälte PartG mbB

(57) **Abstract**

The invention relates to a data processing device (170) and a computer-implemented method for a medical observation device (100), such as a microscope or endoscope. The medical observation device is configured for observing an object (106), which contains at least one fluorophore (116, 118), such as ICG, 5-ALA or is autofluorescence. In order to improve the quality of a reflectance image that is obtained while fluorescence of the at least one fluorophore is recorded, the data processing device is configured to access an input image set (200) comprising at least one digital color input image (130). Each digital color input image represents an image of the object (106). The at least one digital color input image contains a fluorescence-emission signal (222) which is representative of emitted fluorescence of at least one fluorophore in the object. The at least one digital color input image (130) further contains a set of reflectance signals (202). Each reflectance signal represents a reflectance image of the object (106) in a different wavelength band. The set of reflectance signals contain at least a fluorescence-excitation reflectance signal and a supplementary reflectance signal. The fluorescence-excitation reflectance signal (206) is representative of light reflected off the object (106) at wavelengths in the fluorescence excitation spectrum of the at least one fluorophore. The supplementary reflectance signal (204) is representative of light reflected off the object in a wavelength band which is spaced apart from the wavelengths represented in the fluorescence-excitation reflectance signal and the fluorescence-emission signal. The data processing device is then configured to generate a digital reflectance color output image (160) of the object from a combination of the reflectance signals.

## Description

The invention relates to a data processing device and a computer-implemented method for a medical observation device, such as a microscope or an endoscope, for observing an object which contains a fluorophore. The invention further relates to a medical observation device, such as a microscope or endoscope and to a method of observing an object containing at least one fluorophore.

It is often desired to obtain a reflectance image of an object containing a fluorescing fluorophore at the same time as obtaining a fluorescence image of the fluorescing fluorophore. Such a situation arises for example in surgery, where fluorophores mark specific tissues to be more easily recognized by a surgeon. For example, a fluorophore may mark a tumor, which is to be excised, while another fluorophore may mark blood vessels, which are to be avoided during surgery.

The reflectance image supplements the fluorescence image in that it provides the surgeon with a naturally looking image of the surrounding area of the fluorescing fluorophore. The reflectance image thus provides information to the surgeon about the surrounding anatomy.

However, obtaining the reflectance image and the fluorescence image simultaneously is problematic for several reasons. In order to provide a naturally looking reflectance image, it is desired to illuminate the object with white light, such as a standard illuminant. Triggering fluorescence of the fluorophore, however, requires illumination of the object with a fluorescence excitation spectrum, which is not white-light and may need a higher intensity than the white-light illumination. Thus, the illumination that is required for simultaneously obtaining a reflectance image and triggers fluorescence will deviate from a standard illuminant and may thus introduce unnatural looking colors.

Further, the fluorescence of the fluorophores has much lower intensity than the light reflected off the object from the white-light illumination. Consequently, the illumination used for the reflectance image should not contain wavelengths of lights in the fluorescence emission spectrum of the at least one fluorophore, otherwise the fluorescence will be barely detectable without extensive signal processing.

Because of all this, a reflectance image recorded simultaneously with a fluorescence image may not render the anatomy faithfully. Interpretation of such an incomplete reflectance image requires experienced surgeons, is tiring and may lead to erroneous interpretations of the anatomy by the surgeon.

Therefore, there is a need to provide reflectance images with improved color accuracy and anatomy rendition when taken at the same time as a fluorescence image.

This need is addressed by a data processing device for a medical observation device, such as a microscope or an endoscope, for observing an object which contains a fluorophore, wherein the data processing device is configured: to access an input image set, the input image set comprising at least one digital color input image representing an image of the object, the input image set containing in the at least one digital color input image
- a fluorescence-emission signal that is representative of emitted fluorescence of the fluorophore in the object, and
- a set of reflectance signals, each reflectance signal representing a reflectance image of the object in a different wavelength band, the set of reflectance signals containing
   = a fluorescence-excitation reflectance signal that is representative of light reflected off the object at wavelengths in the fluorescence excitation spectrum of the fluorophore, and
   = a supplementary reflectance signal which is representative of light reflected off the object in a wavelength band which is spaced apart from the wavelengths represented in the fluorescence-excitation reflectance signal and the fluorescence-emission signal; and
to generate a digital reflectance color output image of the object from a combination of the fluorescence-excitation reflectance signal and the supplementary reflectance signal.

The above need is further addressed by a computer-implemented method for a medical observation device, such as a microscope or an endoscope, for observing an object which contains a fluorophore, the computer-implemented method comprising the steps of: accessing an input image set, the input image set comprising at least one digital color input image representing an image of the object, the input image set containing
- a fluorescence-emission signal which is representative of emitted fluorescence of the fluorophore in the object,
- a set of reflectance signals, each reflectance signal representing a reflectance image of the object in a different wavelength band, the set containing
   = a fluorescence-excitation reflectance signal which is representative of light reflected off the object at wavelengths in the fluorescence excitation spectrum of the fluorophore,
   = a supplementary reflectance signal which is representative of light reflected off the object in a wavelength band, which is spaced apart from the wavelength represented in the fluorescence-excitation reflectance signal and the fluorescence-emission signal; and
generating a digital reflectance color output image of the object from a combination of the fluorescence-excitation reflectance signal and the supplementary reflectance signal.

The above device and method allow to generate an improved reflectance signal already from just a single digital color input image, which contains both reflectance and fluorescence information. The fluorescence information is contained in the fluorescence-emission signal, which represents the fluorescence of the fluorophore, whereas the reflectance information about the object is contained in the set of reflectance signals, namely the fluorescence-excitation reflectance signal and the supplementary reflectance signal. Using the supplementary reflectance signal in addition to the fluorescence-excitation reflectance signal improves the available information about the reflectance of the object and thus allows to reconstruct a more accurate and reliable reflectance image.

The above data processing device and the above computer-implemented method may be further improved by any of the features, which are described in the following. The features may be used in any combination. Each of the following features is independent of the other features and advantageous of its own. Further, each of the following features may be used for improving the data processing device and/or for improving the computer-implemented method, independent of whether a feature has been described solely within the context of the data processing device or solely in the context of the computer-implemented method. For example, a feature, which is described as a process step, may be used for improving the data processing device simply in that the data processing device is configured to perform this process step. Similarly, a feature of the data processing device may be used for improving the computer-implemented method in that the function performed by this feature is used as a processing step.

For example, it may be preferred that the wavelengths of the emitted fluorescence represented in the fluorescence-emission signal and the wavelengths of the reflected light represented in the excitation-reflectance signal do not overlap, so that there is no crosstalk between these two signals. This facilitates extraction and/or separation of the two signals in the at least one digital color input image.

Further, one or more fluorophores may be contained in the object. Any of these fluorophores may be a fluorophore that is naturally present in the object and/or any of these fluorophore may have been introduced into the object. For example, the at least one fluorophore may be a certain type of material that is naturally present in the object and exhibits (auto)fluorescence if excited with the proper fluorescence excitation spectrum. As another example, the at least one fluorophore may be a fluorophore such as ICG and/or 5-ALA, which may have been injected into the patient or applied to the surgical wound.

Each of the digital color input images of the input image set may consist of or comprise a plurality of pixels. The color of each pixel may be represented by the color space coordinates of a color space. Such a color space may be, for example, an RGB color space. Each color space coordinate may represent a different color, in particular a different primary color. In an RGB color space, there are, for example, three color space coordinates, also termed color bands, R, G, B, which represent the red (R) the green (G) and the blue (B) primary color or color band. There are different RGB color spaces, all depending on the RGB color model. Other color spaces, such as HSV, CIELAB, CIEXYZ or NCS color spaces may be used instead of RGB color spaces.

The digital reflectance color output image is preferably represented in the same color space as the at least one digital color input image. The digital reflectance color output image may be of the same or of a different format than the at least one digital color input image. It may contain the same or a different number of pixels. The same holds if there is more than one digital color input image. In this case, the digital color input images may have the same format and/or size or by of different sizes and/or formats. Independent thereof, the digital color input images may be represented in the same color space or in different color spaces.

For processing purposes, the digital color input images may all be registered and/or converted into the same size, format and/or color space. Further, the digital input images may all have been recorded with the same field of view, the same magnification and/or the same perspective.

Preferably, the digital reflectance color output image is generated in real time. Thus, if a time-sequence of input image sets is generated, e.g. by a frame rate of at least one camera, the generation of the digital reflectance color output image takes preferably less time than the time between two subsequent image sets.

To obtain the reflectance signals, the object may be illuminated simultaneously by at least two different narrow-band spectra. A first one of the two illuminating narrow-band spectra may e.g. be located in the fluorescence-excitation spectrum of the fluorophore. The first illuminating narrow-band spectrum provides both a reflectance image of the object in the fluorescence-excitation spectrum and triggers the fluorescence of the fluorophore. It thus creates the fluorescence-emission signal and the fluorescence-excitation reflectance signal. An example of such an illumination is for example narrow-band blue illumination, for example in the fluorescence excitation band of 5-ALA, which represents the fluorescence-excitation signal.

A second one of the two illuminating narrow-band spectra is preferably spaced apart from the fluorescence-excitation spectrum and the fluorescence-emission spectrum to avoid crosstalk with the light in these two spectra. The light reflected off the object in the second illuminating narrow-band spectrum is represented by the supplementary reflectance signal.

An example of a narrow-band illumination for generating the supplementary reflectance signal may be a narrow-band illumination of the object in the NIR (near infrared) range. The NIR range may extend in one embodiment from about 700nm to about 1100nm, more specifically from about 750nm to about 800nm. In another embodiment, it may comprise IR-A and IR-B and extend from about 700 nm to 3000 nm.

Instead or in addition to illuminating the object using separate narrow-band illumination spectra, optical band-pass filters may be used. For example, the object may be illuminated in a wide spectral band including NIR and recorded using a narrow optical passband which limits the light recorded to the NIR range.

Both the, e.g. blue, fluorescence-excitation reflectance signal and the supplementary, e.g. NIR, reflectance signal can be separated easily from one another as their wavelengths are spaced far from each other. In addition, the NIR range is also sufficiently remote from the fluorescence-emission spectrum, so that the supplementary reflectance signal and the fluorescence-emission signal can also be separated concisely.

As the digital reflectance color image is generated from a combination of the fluorescence-excitation reflectance signal and the supplementary reflectance signal, the fluorescence-emission signal should not be contained in the digital reflectance color output image. Thus, the reflectance signals may need to be separated from the fluorescence-emission signal or, equivalently, extracted from the at least digital color input image before generation of the digital reflectance color output image. For separation and/or extraction of at least one of the reflectance signals from the fluorescence-emission signal, spectral unmixing and/or linear transformation may be applied to the at least one digital color input image.

Each of the reflectance signals is independent of the color space and may be represented by more than one color space coordinate. However, in a very simple case, when the reflectance signal is located in a color band in which the other reflectance and fluorescence-emission signals have very low intensity, the color space coordinate representing this color band may already provide a good approximation of this reflectance signal. It therefore may suffice to extract this color space coordinate, e.g. at a pixel, some or all pixels of the at least one digital color input image, to obtain this reflectance signal.

The set of reflectance signals, in one embodiment, may comprise a second supplementary reflectance signal, which is representative of light reflected off the object in a second wavelength band, which is spaced apart from the wavelengths represented in the fluorescence-excitation reflectance signal and the fluorescence-emission signal and from the wavelength band of the supplementary reflectance signal.

The second supplementary reflectance signal preferably represents a reflectance image of the object in a narrow wavelength band that corresponds to the fluorescence-emission spectrum of a second fluorophore. This allows using the optical filter settings of a medical observation that is configured for observing the fluorescence of two fluorophores by providing a pass-band in the fluorescence-spectrum of the second fluorophore. However, instead of fluorescence, the pass-band is used for recording reflectance. The quality of the second supplementary reflectance signal may be improved if the object is illuminated by a third narrow-band spectrum, which is located in the fluorescence-emission spectrum of the second fluorophore. Thus, the wavelength band of the second supplementary reflectance signal, which would otherwise be used to obtain fluorescence light, is used to supplement the reflectance signal.

The second supplementary reflectance signal may be contained in a digital color input image, which does not contain the fluorescence-emission signal.

In one embodiment, the fluorescence-excitation reflectance signal may be representative of reflected light in the fluorescence-excitation spectrum of 5-ALA, whereas the second supplementary reflectance signal may be representative of reflected light in the fluorescence-excitation spectrum of ICG, or vice versa.

If a second supplementary reflectance signal is used, the digital reflectance color output image is preferably generated from a combination of the fluorescence-excitation reflectance signal, the second supplementary reflectance signal and the supplementary reflectance signal. Using the second supplementary reflectance signal adds further information to the digital reflectance color input in another wavelength band and thus renders the reflectance image of the object more accurate.

The second supplementary reflectance signal may be located in color band, which is different from the color bands in which the fluorescence-excitation reflectance signal, the fluorescence-emission signal and the supplementary reflectance signal are located.

If, for example, 5-ALA is used as the sole fluorophore in the object, the fluorescence-excitation signal is primarily located in the blue color band, whereas the fluorescence-emission signal is located primarily in the red color band. If the supplementary reflectance signal represents the reflectance of the object in the NIR range, then it is advantageous to locate the second supplementary reflectance signal in the green color band, as would be the case if the supplementary reflectance signal is located in the fluorescence-emission spectrum of ICG. In this case, the reflectance signals are located in three different color bands of RGB space and thus represent already a very good approximation to the white-light reflectance of the object.

If the fluorescence-emission signal and any one of the reflectance signals are contained in the same digital color input image, it is preferred that the data processing device is configured to extract the at least one reflectance signal from the color input image or to separate the fluorescence-emission signal from the at least one reflectance signal, e.g. by at least one of spectral unmixing, linear transformation and extraction of a color space coordinate may be used prior to generating the digital reflectance color output image. The extraction allows to separate the reflectance signals from the fluorescence-emission signal and thus to process the reflectance information separately from the fluorescence information.

As already mentioned above, the extraction and/or separation of a reflectance signal may be as simple as extracting a color space coordinate, for example the blue, green or red color space coordinate, depending on in which color band the reflectance signal to be extracted is located.

The fluorescence excitation spectrum may be in the visible blue and/or green color band. The blue color band may comprise or consist of light having wavelengths between around 450nm and about 490nm. The green color band may consist of or comprise light having wavelengths between about 490nm and about 570nm.

Preferably, the wavelengths in the fluorescence excitation spectrum are between about 400nm and about 480nm, more preferably between about 420nm and about 450nm.

The fluorescence-emission signal may comprise light comprising and/or consisting of light in the red color band. The red range may comprise or consist of light of wavelengths between about 650nm to about 700 or 750nm.

For example, if the reflectance color output image is represented in RGB color space, the blue color band may in one embodiment comprise or consist of the reflectance signal and the red color band may comprise or consist of the supplementary reflectance signal.

The supplementary reflectance signal may be representative for light reflected off the object in the NIR range. The supplementary reflectance light is, according to another aspect preferably located in the NIR range. The light reflected off the object in the NIR range surprisingly is a very good substitute for the light reflected by the object in the visible red and therefore is able to create a very natural looking red in the digital reflectance color output image. This is particularly important in surgical applications where red is a dominant color and provides important information to the surgeon about the surgery.

Using the NIR range as the supplementary reflectance signal is particularly useful if the fluorescence-emission signal is in the red color range but does not extend into the NIR range. If there is no or hardly any overlap, the fluorescence-emission signal may be more easily separated from the supplementary reflectance signal, even if both are in the same color band, using optical filters and/or spectral unmixing. In this case, the extracted supplementary reflectance signal in the NIR range may be used as a surprisingly good approximation for the red component of the white-light reflectance image of the object if no fluorescence-emission signal is in the red color band.

The at least one digital color input image is recorded with at least one color camera. Typically, such a color camera may comprise, at each pixel, a different sensor for each color band. Each of these sensors has a sensitivity curve, which may not be strictly limited to the respective color band but may extend to at least on other color band. In an RGB camera, the sensitivity curves of the R, G and B color space coordinates may not be separate from each other but may overlap. Thus, light in one color band may have non-zero color space coordinates in the other color bands. These non-zero color space coordinates provide additional information that is useful in the extraction and/or separation of the reflectance signals when spectral unmixing and/or linear transformation is applied.

In one embodiment, the data processing device may be configured to extract, prior to generating the digital reflectance color image, at least two, for example, two or three reflectance signals from two or three digital color input images of the input image set simultaneously. Using more than one digital color input image for the extraction and/or separation may increase the accuracy of the separation and/or extraction process, as there is more information encoded in a plurality of digital color input images than in a single digital color input image.

In one embodiment, the data processing device may be configured to apply a linear transformation to at least one reflectance signal extracted by spectral unmixing. For example, the fluorescence-excitation reflectance signal, the supplementary reflectance signal and, optionally, the second supplementary reflectance signal may be extracted by spectral unmixing. To the extracted signals, a linear transformation may be applied either in individually or in any combination. The linear transformation for example may represent a color correction function, which represents a previously performed color calibration.

In one example, the reflectance signals extracted at each pixel of the at least one digital color input image may be combined to form an intermediate reflectance color image to which the linear transformation is applied either pixel-by-pixel or to the entire image. This combination may comprise forming a union set of the set of color space coordinates of corresponding pixels of the digital color input images. If, for example, at each pixel of the digital color input images, three color space coordinates are provided and three reflectance signals are used for generating the digital reflectance color output image, then the union set comprises nine color space coordinates at this pixel. If two such reflectance signals are used, then the union set comprises six color space coordinates. The linear transformation is then preferably carried out on the union set.

Corresponding pixels are pixels in two images of an object that represent the same location of the object, preferably at the same scale and in the same perspective. Corresponding pixels may be located at the same position within the images, if the two images are registered. Otherwise, corresponding pixels are determined by features analysis and located at the same position of the imaged feature.

The linear transformation may be dependent on the color space coordinates of the pixel, so that a different linear transformation is applied to different pixels depending on their color. Thus, color conversion may be used to correct different colors differently. Here, the term color refers to a combination of all color parameters, such as hue, intensity, saturation.

The linear transformation may, in one embodiment, comprise a multiplication of the at least one color input image, which contains the reflectance signals for generation of the digital reflectance color image, by a color conversion matrix.

The color conversion matrix preferably has two dimensions. One of the two dimensions corresponds to the number of color space coordinates in the digital reflectance color output image. The other one of the two dimensions of the color conversion matrix corresponds to the total number of color space coordinates contained in the digital color input images from which the at least one reflectance signal is to be extracted.

If, in this case, the digital reflectance color output image is represented in an RGB color space, the one dimension of the color conversion matrix will be three.

If the reflectance signals are to be extracted from one digital color input image, which is represented in an RGB color space, the other dimension of the color conversion matrix will also be three. If two digital color input images are used, the other dimension of the color conversion matrix will be six. If three digital color input images are used for extraction, the other dimension of the color conversion matrix will be nine.

The digital reflectance color output image may be directly obtained by applying the color conversion matrix to the digital color input images, which contain the fluorescence-excitation reflectance signal, the supplementary reflectance signal and, optionally, the second supplementary reflectance signal.

If the color conversion matrix is applied to reflectance signals that have been extracted from the respective digital color input images by spectral unmixing, the other dimension of the color conversion matrix corresponds to the total number of color space coordinates in the reflectance signals that were extracted by spectral unmixing. In this case, each extracted reflectance signal is treated as a digital color input image to the linear transformation or the multiplication with the color conversion matrix respectively.

If the reflectance signals are extracted by extracting a separate color space coordinate for each reflectance signal, the digital reflectance color output image may be formed by simply combining the extracted color space coordinates. If, for example, the fluorescence-excitation reflectance signal is extracted by extracting the blue color space coordinate, the supplementary reflectance signal is extracted by extracting the red color space coordinate and optionally the second supplementary reflectance signal is extracted by using the green color space coordinate, the digital reflectance color output image may be obtained in that each different reflectance signal corresponds to a different color space coordinate of the digital reflectance color output image.

Still, even in this case, it is preferred that a linear transformation, in particular an application of a color conversion matrix takes place after the formation of the digital reflectance color output image from the reflectance signals. Such a linear transformation may be used to obtain more natural looking colors.

A plurality of different color conversion matrices may be provided, e.g. stored in the data processing device. The color conversion matrix may be selected automatically depending on the color of the pixel to which the color conversion matrix is to be applied.

According to a further aspect, the input image set may comprise a digital fluorescence-light color input image as a digital color input image of the input image set. Such a digital fluorescence-light color input image is recorded, for example, by a digital fluorescence color camera of the medical observation device. The digital fluorescence-light color camera is preferably configured to record the fluorescence emission of the at least one fluorophore. The digital fluorescence-light color camera may also be configured to record at least part of the fluorescence-excitation spectrum. The digital fluorescence-light color camera may alternatively or cumulatively be configured to record the supplementary reflectance signal.

In particular, the digital fluorescence-light color input image may contain at least two reflectance signals of the group of reflectance signals containing the supplementary reflectance signal, the fluorescence-excitation reflectance signal and the second supplementary reflectance signal.

The digital fluorescence-light camera may e.g., be provided with a fluorescence filter set that allows to pass only the fluorescence emission spectrum of at least one fluorophore of which fluorescence is to be recorded. Additionally or alternatively, the fluorescence filter set may be configured to pass at least part of the fluorescence-excitation spectrum, so that the digital fluorescence-light color camera may record the fluorescence-excitation reflectance signal. Additionally, the fluorescence filter set may comprise a supplementary pass band that contains the wavelengths recorded in the supplementary reflectance signal. The supplementary pass band is preferably located in the NIR.

The data processing device may be configured to extract the supplementary reflectance signal from the fluorescence-light digital color input image prior to generating the digital reflectance color image.

According to another aspect, the digital fluorescence-light color input image may contain the fluorescence-excitation reflectance signal and the data processing device may be configured to extract a fluorescence-excitation reflectance signal from the fluorescence-light digital color input image prior to generating the digital reflectance color image.

According to another aspect, the input image set may comprise a digital white-light color input image as a digital color input image. The digital white-light color input image is provided preferably in addition to the digital fluorescence-light color input image described above.

The digital white-light color input image may contain the fluorescence-excitation reflectance signal, in particular if the fluorescence-excitation reflectance signal is not contained in the digital fluorescence-light color input image. The data processing device is then preferably configured to extract the fluorescence-excitation reflectance signal from the digital white-light color input image prior to generating the digital reflectance color output image.

In another embodiment, the digital white-light color image may contain in particular at least one reflectance signal of the group of reflectance signals containing the supplementary reflectance signal, the fluorescence-excitation reflectance signal and the supplementary reflectance signal, wherein at least one reflectance signal of the group is not contained in the digital fluorescence-light color input image.

The digital white-light color input image may be recorded by a digital white-light color camera of the medical observation device. The digital white-light color camera may be provided with a white-light filter set having pass-bands that preferably do not overlap the pass-bands of the fluorescence filter set.

The second supplementary reflectance signal may be contained in the digital white-light color input image or the digital fluorescence-light color input image. In particular, the second supplementary reflectance signal may be contained in the digital white-light color input image.

The input image set, in a further embodiment, may comprise a second digital fluorescence-light color input image, which contains the second supplementary reflectance signal. The medical observation device may comprise a second fluorescence light camera for recording the second digital fluorescence-light color image. The second digital fluorescence-light color camera may comprise a second fluorescence filter set having a pass-band that is configured to transmit preferably only light in the fluorescence-emission spectrum of a second fluorophore. The second digital fluorescence-light camera does not need to be a color cameral, but preferably is so. If the second fluorescence color camera is a black and white camera, the black and white image may simply be assigned to a color space coordinate, which corresponds to the color of the fluorescence of the second fluorophore.

In particular, the pass-bands of the white-light filter set, the fluorescence filter set and, if present, the second fluorescence filter set may be complementary to one another and in combination may cover the entire visible light range and, if necessary, also the NIR range.

The data processing device may be configured to extract the second supplementary reflectance signal from the respective digital color input image, in particular the second digital fluorescence-light color input image.

Instead of just extracting a reflectance signal from the white-light digital color input image, the entire white-light digital color input image may be used as a reflectance signal in another embodiment. This may alleviate the computational burden on the data processing device as an extraction of the reflectance signal is not needed.

The data processing device comprising any of the above features and/or configured to carry out any of the above-described process steps may be part of the medical observation device. The data processing device may comprise hardware and/or software elements. The data processing device may be an embedded processor of the medical observation device. The embedded processor is also used as a controller of the medical observation device. Alternatively, the data processing device may be part of a computer system, which is part of the medical observation device. The data processing device may also be distributed across a plurality of processors, which may reside in different entities.

Further, the medical observation device may comprise a second digital fluorescence-light camera configured to record the second supplementary reflectance signal. For generating the digital reflectance color output image, the second digital fluorescence-light camera, however, does not capture the fluorescence of the second fluorophore but the reflectance of the object in the fluorescence-emission spectrum of the second fluorophore.

The medical observation device may comprise an optical observation filter assembly configured to filter the light from the object before arriving at at least one of the digital fluorescence-light cameras, the second digital fluorescence-light camera and the digital white-light camera. The white-light filter set and the at least one fluorescence filter set may be comprised by the optical observation filter assembly.

The optical observation filter assembly may comprise a plurality of different, in particular non-overlapping, pass bands. The pass bands may complement each other by preferably covering particularly the visible range completely and at least part of the NIR spectrum if the NIR spectrum is used.

In particular, the plurality of pass bands may comprise a first pass band configured for transmission of light in the fluorescence spectrum of the at least one fluorophore; a second pass band configured for transmissions of light in the fluorescence-excitation light of the at least one fluorophore; a third pass band configured for transmission of light having wavelengths larger than 700nm, wherein the third pass band may be located entirely in the NIR range; a fourth pass band configured for transmissions of the fluorescence of another fluorophore, the fourth pass band spaced apart from and/or non-overlapping the second pass band, wherein the fourth pass band may be configured for transmissions of light located preferably entirely in the green light range; and/or a fifth pass band configured for transmission of visible light except light in wavelengths that are located in at least one of the second, third and fourth pass band.

At least one of the first, second and third pass bands may be located in front of the digital fluorescence-light color camera, but preferably not in front of any other camera of the medical observation device. In particular, the first, second and third pass band may be located in the optical path between a beam splitter and the digital fluorescence light color camera.

At least one of the second, third and fifth pass bands may be located in front of the digital white-light color camera, but not in front of another camera of the medical observation device. Again, at least one of the second, third and fifth pass bands may be located in particular in the optical path between a beam splitter and the digital white-light color.

The fifth pass band may be located in front of the second digital fluorescence-light color camera if present. The fifth pass band then is preferably not located in front of any other camera of the medical observation device.

Additionally or alternatively to the observation filter assembly, the medical observation device may comprise an illumination assembly which is configured to illuminate the object simultaneously with light in discrete wavelength bands. In particular, the discrete wavelength bands may correspond to the first, second, third, fourth and/or fifth pass-band of the observation filter assembly.

For example, the illumination assembly may be configured to illuminate the object with light in a (discrete) wavelength band that is contained in the excitation spectrum of a fluorophore. Illumination in this wavelength band reflected off the object is represented in the fluorescence-excitation reflectance signal.

The illumination assembly may be further configured to illuminate the object in a (discrete) wavelength band that corresponds to the wavelength of the supplementary reflectance signal. Preferably, the illumination assembly may be configured to illuminate the object in a wavelength band that starts at about 700 nm or is entirely located in the NIR range. Illumination in this wavelength band reflected off the object is represented in the supplementary reflectance signal.

Further, the illumination assembly may be configured to illuminate the object with light in a (discrete) wavelength band that is contained in the fluorescence-emission spectrum of another fluorophore, i.e. not the fluorophore of which fluorescence is triggered by illumination in the fluorescence-emission spectrum. Illumination in this wavelength band reflected off the object is represented in the second supplementary reflectance signal.

For illuminating the object in these discrete wavelength bands, the illumination assembly may comprise one or more, in particular, tunable and/or exchangeable optical filters, in particular band-pass filters, and/or a tunable light source. The illumination assembly may further be configured to provide simultaneous illumination of the object in a plurality of discrete wavelength bands or discrete illumination spectra that are preferably non-overlapping, spaced apart from one another and/or narrow band.

In particular, the illumination assembly may be configured to provide an illumination spectrum covering the entire visible light range plus optionally the NIR range. Further, the illumination assembly may be configured to generate an illumination spectrum that corresponds to a standard illuminant.

The medical observation device in another embodiment may be configured to illuminate the object with NIR light in the NIR range; wherein the NIR light reflected off the object is represented by the supplementary reflectance signal, and wherein the data processing device is configured to generate at least the color space coordinate representing red light in the digital reflectance color output image depending on the supplementary reflectance signal, in particular solely depending on the supplementary reflectance signal. The medical observation device may be configured to record the light reflected in the NIR range with one of the digital fluorescence-light color cameras and the second digital fluorescence-light color camera.

In another wording, the illumination assembly is configured to illuminate the object simultaneously in at least two of the group of discrete wavelength bands comprising:
- a first discrete wavelength band located preferably entirely in the fluorescence-emission spectrum of a fluorophore;
- a second discrete wavelength band located preferably entirely in the fluorescence-excitation spectrum of another fluorophore;
- a third discrete wavelength band comprising wavelengths larger than 700 nm and/or located entirely in the NIR range.

The invention further relates to a method of observing an object containing at least one fluorophore, wherein the method comprises the computer-implemented method in any of the above-described configurations and the step of recording a digital fluorescence-light color image as a digital input image, the digital fluorescence-light color image containing the fluorescence-emission signal and at least one of the reflectance signals.

Finally, a computer program product or computer-readable storage medium may be provided comprising instructions, which, when executed by a computer, cause the computer to carry out the computer-implemented method in any of the above configurations.

In the following, the invention is described exemplarily with reference to various examples and with reference to the drawings. The combination of features that are shown in the embodiments is not to be considered as limiting. For example, features of the embodiments, which have a technical effect as e.g. explained above, that is not needed in a specific application, may be omitted. Conversely, features described above that are not part of an embodiment described below may be added if the technical effect associated with this particular feature is needed in a specific application.

Throughout the description and the drawings, the same reference numerals are used for elements that correspond to each other with respect to function and/or structure.

In the drawings,
- Fig. 1: shows a schematic representation of a medical observation device for generating a digital reflectance color output image from at least one digital color input image;
- Fig. 2: shows a schematic representation of the generation of a digital reflectance color output image from reflectance signals contained in two digital color input images;
- Fig. 3: shows a schematic representation of the spectrum of a digital reflectance color output image generated from two digital color input images;
- Fig. 4: shows a schematic representation of the spectrum of a digital reflectance color output image generated from a single digital color input image;
- Fig. 5: shows a schematic representation of the spectrum of a digital reflectance color output image generated from two digital color input images;
- Fig. 6: shows a schematic representation of the spectrum of a digital reflectance color output image generated from a single digital color input image;
- Fig. 7: shows a schematic representation of the spectrum of digital reflectance color output image generated from three digital color input images;
- Fig. 8: shows a schematic representation of a linear transformation for generating the digital reflectance color output image;
- Fig. 9: shows an overview for generating a digital reflectance color output image;
- Fig. 10: shows a schematic representation of a generic medical observation device.

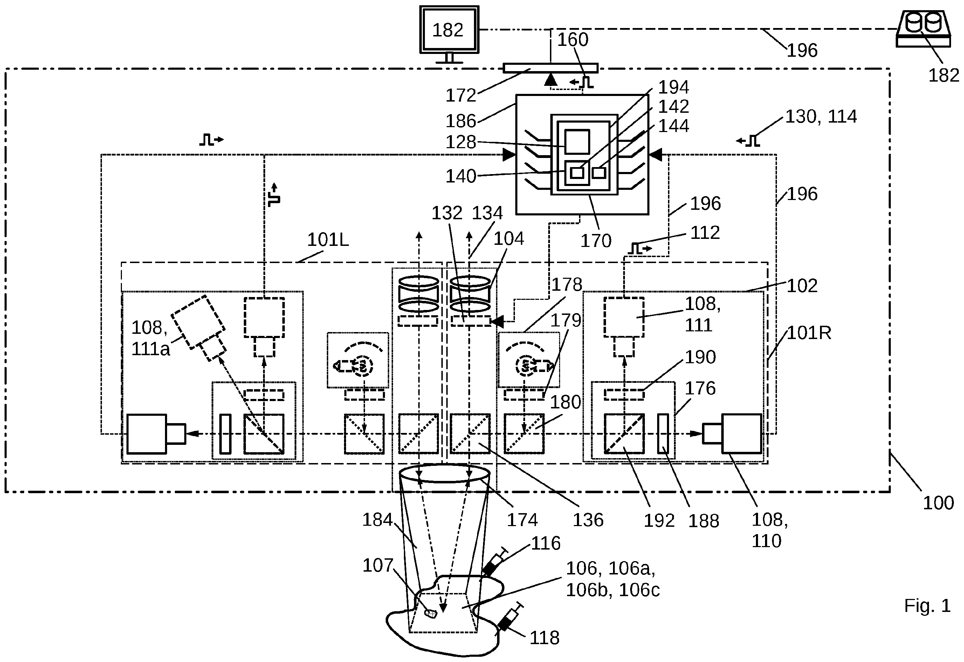

Fig. 1 shows schematically a medical observation device 100. The medical observation device 100 may be a microscope or an endoscope, the difference between a microscope and an endoscope being primarily that, in an endoscope (not shown), an object 106 is viewed through optical fibers that are brought into vicinity of the object 106 to be investigated, e.g. by insertion into a body containing the object, whereas, in a microscope, an objective 174 is directed onto the object. Although the medical observation device of Fig. 1 is a microscope, the following description also applies to an endoscope.

The medical observation device 100 may be a medical observation device used in surgery. The medical observation device 100 may also be a medical observation device used in a laboratory, such as a laboratory microscope. The object 106 to be investigated may consist of or comprise biological tissue 107.

The object 106 may contain one or more fluorophores 116, 118. The at least one fluorophore may be a fluorophore that is naturally contained in the object. For example, bone and blood contain fluorophores. The at least one fluorophore may also be added to the object 106, e.g. by injecting it into the biological tissue 107. Examples of fluorophores that may be added to the object 106 are ICG, fluorescein and/or 5-ALA.

The medical observation device 100 as shown is a fluorescence imaging device. Thus, the medical observation device is configured to view and preferably also excite the fluorescence of the one or more fluorophores 116, 118.

The medical observation device 100 may be a stereoscopic device as is exemplarily shown in Fig. 1. It may thus comprise two identical subassemblies 101L and 101R for each of the two stereoscopic channels. As the two subassemblies 101L, 101R are identical with respect to function and structure, the following description focuses on the right subassembly 101R, but applies identically to the left stereoscopic channel 101L.

The medical observation device 100 may alternatively be a monoscopic device. In this case, only one of the two subassemblies 101L, 101R may be present. For a monoscopic medical observation device 100, the following description therefore applies as well.

The medical observation device 100 in operation provides an input image set of digital color input images 130 that are processed by a data processing device 170. The data processing device 170 may be an integral part of the medical observation device 100. In one example, it is a processor which is embedded in the medical observation device and also used as a controller for controlling the hardware of the medical observation device 100. In another example, the data processing device 170 is part of a general computerwhich is connected to the medical observation device for uni- or bi-directional data transfer by wire or wirelessly.

The input image set of digital color input images 130 may comprise one or more digital fluorescence-light color input images 112 and a digital white-light color input image 114.

The digital white-light color image 114 preferably represents a reflectance image of the object 106 in the visible light range. The visible light range or visible spectrum comprises the wavelengths from about 310 nm to about 1100 nm, or from about 380 nm to 750 nm, or from about 450 nm to about 700 nm. The fluorescence spectrum or, if more than one fluorophore is used, the fluorescence spectra of the fluorophores 116, 118 is preferably omitted from the spectrum recorded in the digital white-light color input image 114. This makes sure that only or predominantly reflected light is contained in the digital white-light color input image 114 if fluorescence is present. For example, if 5-ALA is used as a fluorophore, the fluorescence spectrum from about 625 nm to about 650 nm may not be recorded in the digital white-light color input image 114.

If light of specific wavelengths is used to excite fluorescence of the at least one fluorophore, the spectrum comprising or consisting of these wavelengths may be recorded or represented in the digital white-light color input image 114. For example, if 5-ALA is used as a fluorophore, fluorescence may be excited by illuminating the object 106 with wavelengths between about 380 nm to about 450 nm, i.e. in the blue light range. For fluorescein and ICG and other fluorophores, different but known ranges for the excitation and emission spectra apply than for 5-ALA. In the application described herein, the light reflected off the object by the light exciting fluorescence is recorded in the digital white-light color input image 114. The light in the excitation spectrum provides an illumination of the object 106 and is reflected off the object 106. The reflected excitation spectrum provides reflectance information about the object 106 in form of a fluorescence-excitation reflectance signal.

Thus, the digital white-light color input image 114 represents the reflectance of the object 106.

If the illumination light is white light, the digital white-light color input image 114 represents a white-light image of the object 106 as it would be seen by a human observer. In this case, the digital white-light color input image 114 may be regarded as a natural color or true-color input image. If the fluorescence excitation light is recorded, the digital white-light color input image 114 rendered in natural colors will-to the human observer-represent a highly tinted image of the object 106 due to the narrow-band characteristics of the fluorescence excitation light.

The digital fluorescence-light color input image 112 may represent the fluorescence emission of the one or more fluorophores 116, 118. In particular, the fluorescence emission may be triggered by the fluorescence excitation light that may be recorded in the digital white-light color input image 114. The fluorescence emission information is represented in the digital fluorescence-light color input image 112 in the form of a fluorescence-emission signal.

If there is more than one fluorophore 116, 118 in the object 106, the emission spectrum of the different fluorophores may be recorded, by different digital fluorescence-light color input images 112, respectively. For example, the emission spectrum of 5-ALA/pPiX as one fluorophore may be recorded in one digital fluorescence-light color input image 112 and the emission spectrum of ICG as another fluorophore may be recorded in another digital fluorescence-light color input image 112. Of course, the fluorescence signals or images of 5-ALA/pPiX and ICG may also be recorded in a single digital fluorescence-light color input image, and be separated using image processing such as spectral unmixing.

In the embodiment as shown, a wavelength band outside the emission spectrum or the emission spectra of the one or more fluorophores and outside the fluorescence excitation spectrum of the at least one fluorophore 116, 118 may be represented in one of the digital white-light color input image 114 and the digital fluorescence-light color input image 112-preferably the latter. As there is no fluorescence in this wavelength band, a reflectance image of the object will be recorded in this wavelength band, which may, for example, be located in or comprise NIR wavelengths. The reflectance image in this additional, or supplementary, wavelength band forms a supplementary reflectance signal in the digital fluorescence-light color input image 114. The fluorescence-emission signal and the supplementary reflectance signal may be separated from one another-or be individually extracted from the digital fluorescence-light color input image 114 as is described in more detail further below. The NIR wavelengths are 700 nm to 1100 nm, preferably 750 nm to 800 nm.

The digital color input images 130 are color input images comprising a plurality of pixels. They are recorded using color bands or, equivalently, primary colors of a color space. The color space comprises at least three of such color bands. In the color space, each color band is represented by a different color space coordinate. For conversion between different color spaces, color space transformations may be used. In a different color space, the same color is represented in different color space coordinates. Each pixel of the digital color input images 130 comprises a set of color space coordinates that together represent the color of the respective pixel. Each color band thus may be regarded as representing a color space axis.

For example, the digital color input images 130 may be recorded in RGB color space using the three primary colors or color bands-or color space coordinates-R, G, B. Alternatively, the digital color input image 130 may be recorded in different color spaces, respectively, and/or represent multi-spectral or hyperspectral color input images. The digital white-light color input image 114 and the digital fluorescence color input image 112 need not be recorded in the same color space, although this is preferred.

In RGB color space, each color is represented by triple of three color space coordinates in the form of integer numbers, wherein each integer number indicates the intensity of one of the primary colors R, G, B. For example, the most intense red is indicated by the triple [255 ,0, 0]. The most intense green color is indicated by [0, 255, 0], and the most intense blue by [0, 0, 255]. Thus, RGB color space is a three-dimensional space, CMYK color space would be a four-dimensional space. A color can be considered as a point in color space to which a vector such as [0, 0, 255] points. A multispectral or hyperspectral color space having n color bands would correspondingly result in an n-dimensional color space, in which each color is represented by an n-tuple of color space coordinates.

The spectrum recorded in the digital white-light color input image 114 and the spectrum recorded in the at least one digital fluorescence-light color input image 112 are preferably supplementary to one another, i.e. do not overlap.

More specifically, the digital imaging system 102 may comprise at least one digital color camera 108 for generating the input image set of digital color input images 130, for example the at least one digital fluorescence-light color input image 112 and the digital white-light color input image 114. Specifically, the digital imaging system 102 may comprise a digital white-light color camera 110 and one or more digital fluorescence-light color cameras 111, 111a. The second digital fluorescence-light color camera 111a is optional. In Fig. 1, the second digital fluorescence-light color camera 11a is only shown in the left stereoscopic channel 101L, but of course may be present in the right stereoscopic channel 101R. Alternatively, the digital fluorescence-light camera of one stereoscopic channel may be used as the (first) digital fluorescence-light color camera 111 and the digital fluorescence-light camera of the other stereoscopic channel may be used as the second fluorescence-light camera 111a.

The white-light color camera 110 is configured to record the digital white-light color input image 114. In particular, the white-light color camera 110 may be configured to generate a stream of digital white-light color input images 114 in the form of a video stream. The white-light color camera 110 is preferably configured to record a digital image across the entire visible spectrum in the wavelengths indicated above. The white-light color camera 110 may be a CCD, CMOS or multispectral or hyperspectral camera.

The at least one fluorescence-light color camera 111 is configured to record the digital fluorescence-light image 112. In particular, the fluorescence-light camera 111 may be configured to generate a stream of digital fluorescence-light color input images 112 in the form of a video stream. The fluorescence-light color camera 111 may be configured to record the digital fluorescence-light color input image 114 only in the fluorescence spectrum or the fluorescence spectra of the at least one fluorophore 116, 118. The fluorescence-light camera 111 may be configured to record the digital fluorescence-light image only in one or more narrow bands of light. These narrow bands should overlap the fluorescence spectrum or spectra of the one or more fluorophores 116, 118 of which fluorescence is to be recorded. Preferably, the fluorescence spectra of the fluorophore 116 and the second fluorophore 118 are at least partly separate, preferably completely separate, i.e. non-overlapping, so that the fluorescence-light camera 111 may record light in two separate fluorescence bands that are spaced from one another. Alternatively, if two fluorescence-light color cameras 111, 111a are provided, each fluorescence-light color camera 111 preferably captures the fluorescence emission of a different fluorophore, thus providing two digital fluorescence-light color input images 112 containing different fluorescence-emission signals.

If a second fluorophore is present in the object 106 and the object 106 is illuminated with the excitation spectrum of the second fluorophore in order to trigger its fluorescence, the light reflected off the object in the excitation spectrum of the second fluorophore may be used to gain additional information about the object 106. This information is contained in a second supplementary reflectance signal that may be recorded by any of the cameras 110, 111 and, if present, 111a. The second supplementary reflectance signal may be separated from the other signals contained in the digital color input images 130 or extracted from the digital color input images 130 as is described further below.

The fluorescence-excitation reflectance signal, the supplementary reflectance signal and-optionally-the second supplementary reflectance signal form a set of reflectance signals, each representing the reflectance of the object in different, preferably non-overlapping, spectra or, synonymously, wavelength bands.

The at least one fluorescence-light color camera 111, 111a may be a CCD, CMOS or multi-spectral or hyperspectral camera. Preferably, the white-light color camera 110 and the at least one fluorescence-light color camera 111 are of the same type, although this is not necessary.

The respective fields of view 184 of the cameras 110, 111 and 111a if present are preferably aligned or even coinciding and coaxial. Thus, it is preferred that the cameras 110, 111 provide the identical field of view 184 with the identical perspective and focal length. This results in identical representations of the object 106 in the images 112, 114 generated by the different cameras 110, 111. Both cameras 110, 111 may use the same objective 174.

It is preferred that the cameras 110, 111, and, if present, 111a are operated synchronously. Specifically, the exposure times may be synchronized. Thus, the medical observation device 100 may be configured to generate the digital white-light color input image 114 and the digital fluorescence-light image 112 at the same time.

Preferably, the gain of the at least two cameras 110, 111, 111a is synchronized, i.e. adjusted in the at least two cameras 110, 111, 111a at the same time. Moreover, the ratio of the gain applied in camera 110 to the gain applied in camera 111 and, if present, in camera 111a may be constant, even if the gain is changed. The gamma correction and color adjustment or white balance may be switched off or kept constant.

The data processing device 170 may comprise preprocessing routines 128 that are applied to one or more digital color input image 130. The preprocessing routines 128 may comprise matching or registering routines and/or normalization routines. If, for example, a match of the magnification, perspectives and/or field of view of two or more digital input images 130 cannot be generated optically, such match may be generated using image processing by applying a matching or registering routine to the digital images 112, 114. Further the digital color input images 130 or any signals contained therein may be normalized before they are processed further.

Any of the above measures facilitates the comparison, joint processing and/or combining of the digital color input images 130.

For optically separating the spectrum recorded in the digital white-light color input image 114 from the spectrum recorded in the at least one digital fluorescence-light color input image 112, i.e. for separating the reflectance spectrum from the fluorescence spectrum, an optical color-separation assembly 176 may be provided. The color-separation assembly 176 may comprise optical elements such as a beam splitter 192, which may be dichroic. The color separation assembly 176 may further or alternatively comprise an optical white-light filter 188 and/or an optical fluorescence filter set 190. The optical white-light filter set 188 and the fluorescence-filter set 190 may be part of an observation filter assembly 187.

The fluorescence filter set 190 is preferably configured to transmit light in the fluorescence spectrum or spectra of the one or more fluorophores 116, 118 and to block light outside the fluorescence spectrum or spectra.

The fluorescence filter set 190 may comprise one or more optical band-pass filters comprising one or more passbands. Each passband should overlap the fluorescence emission spectrum of a respective fluorophore 116, 118 of which the fluorescence is to be recorded. As the fluorescence-light filter set 190 is in the light path between the beam splitter 192 and the fluorescence-light color camera 111, only the wavelengths in the passbands of the fluorescence-light filter set 190 are transmitted to the fluorescence-light camera color 111.

If two fluorescence-light color cameras 111, 111a are used to capture different fluorescence emission spectra, the fluorescence filter set 190 may comprise a different optical band-pass filter in front of each of the fluorescence-light color cameras 111, 111a. The pass-band of one band-pass filter may be contained in the fluorescence-emission spectrum of one fluorophore, whereas the pass-band of the other band-pass filter may be contained in the fluorescence-emission spectrum of another fluorophore in the object 106.

The white-light filter 188 is preferably configured to block light in the fluorescence spectrum or spectra of the one or more fluorophores 116, 118. The white-light filter 188 may also be configured to block light in the fluorescence-excitation spectrum.

The white-light filter 188 is preferably configured as a band-stop filter, of which the stop bands correspond to or at least contain the passbands of the fluorescence-light filter set 190. The white-light filter 188 is located in the light path between the beam splitter 192 and the white-light camera 110. Thus, white-light camera 110 records only wavelengths that are outside the stop-bands of the white-light filter 188 and therefore also outside of the pass-bands of the fluorescence-light filter set 190 of the fluorescence-light filter set 190.

Any one of the white-light filter set 188 and the fluorescence filter set 190 may be a tunable filter.

If the beam splitter 192 is a dichroic beam splitter, at least one of the filters 188, set 190 may be omitted as the optical spectral filtering in this case is already integrated in the dichroic beam splitter. The above description of the passbands and stopbands then should apply mutatis mutandis to the dichroic beam splitter 192.

The medical observation device 100 may further comprise an illumination assembly 178, which is configured to illuminate the object 106 preferably through the objective 174 through which the imaging system 102 records the at least one digital image 112, 114.

The illumination assembly 178 may be configured to selectively generate white-light, i.e. light that is evenly distributed across the entire visible spectrum, and fluorescence-excitation light, which contains light only in wavelengths that stimulate fluorescence of the at least one fluorophore 116, 118. The illumination light generated by the illumination assembly 178 may be fed into the objective 174 using an illumination beam splitter 180.

The illumination assembly 178 preferably is configured to generate illumination light simultaneously in a plurality of discrete, in particular narrow-band, wavelength bands.

These wavelength bands may comprise any of or any combination of the following wavelength bands.

One such discrete wavelength band may be entirely located in the fluorescence-excitation spectrum of a fluorophore 116.

Another such wavelength band may be entirely located in the fluorescence-emission spectrum of another fluorophore 118.

Another such wavelength band may be limited to wavelengths larger than 700 nm and be entirely located in the NIR range.

The simultaneous illumination of the object with any of the discrete wavelength bands as described above may be accomplished by a light source 199, e.g. a tunable light source such as a light source comprising a plurality of LEDs in different colors, in particular in different primary colors, which is configured to generate light in these wavelength bands simultaneously. Alternatively or additionally, the wavelength bands may be generated by using an illumination filter 179 having multiple pass-bands, wherein the pass-bands preferably correspond to the above wavelength bands. If such an illumination filter 179 is used, the light source 199 may generate white-light which is then filtered by the illumination filter 179 so that only the light in the pass-bands illuminates the object 106.

The illumination filter 179 may be provided depending on the at least one fluorophore, of which fluorescence is to be triggered, and its specific excitation spectrum. For example, if 5-ALA is used as a fluorophore, the illumination filter may have a transmission of 90 % to 98 % up to wavelengths of 425 nm, a transmission between 0.5 % and 0.7 % in wavelengths between 450 nm and 460 nm, a transmission of not more than 0.1 % between 460 nm and 535 nm and of practically zero for wavelengths above 535 nm. The illumination filter 179 may be configured for pass-through of NIR light. For example, the illumination filter 179 may comprise a pass-band in the NIR. The illumination filter 178 may further comprise a passband, which is preferably entirely located in the fluorescence-excitation spectrum of another fluorophore.

The data processing device 170 may be a hardware module, such as a microprocessor, or a software module. The data processing device 170 may also be a combination of both a hardware module and a software module, for example by using software modules that are configured to be run on a specific processor, such as a vector processor, a floating point graphics processor, a parallel processor and/or on multiple processors. The data processing device 170 may be part of a general-purpose computer 186, such as a PC. In another embodiment, the data processing device 170 is an embedded processor of the medical observation device 100.

The data processing device 170 is configured to retrieve the digital white-light color input image 114 and the digital fluorescence-light image 112. For example, the data processing device 170 may be configured to retrieve the digital white-light color input image 114 and the digital fluorescence-light image 112 from a memory 194 and/or directly from the cameras 110, 111 and if present 111a. The memory 194 may be part of the data processing device 170 or reside elsewhere in the medical observation device 100.

The data processing device 170 is further configured to compute a digital reflectance color output image 160 from the input image set of digital color input images 130, specifically from the signals contained in the input image set of digital color input images 130 that are representative of reflectance images of the object 106 at different spectra. In the embodiment shown in Fig. 1, the fluorescence-emission signal, i.e. the fluorescence of the at least one fluorophore is preferably not contained in the digital reflectance color output image 160.

The digital reflectance color output image 160 is a color input image, which is represented in a color space. The color space of the digital reflectance color output image may be different from the color space of any of the digital white-light color input image 114 and the at least one digital fluorescence-light image 112. Preferably, however, the color space of the digital reflectance color output image 160 is the same color space as that of the digital color input images 130, such as the digital white-light color input image 114 and the at least one digital fluorescence-light image 112.

If all the images 112, 114, 160 are in RGB color space, the dimension of the color conversion matrix is preferably 6x3 or 3x6. If two digital fluorescence-light color input images 114 are used in the generation of the digital reflectance color output image 160, the dimension of the color conversion matrix is 9x3 or 3x9.

The medical observation device 100 may be adjusted to a different fluorophore by re-configuring the color-separation assembly 176, e.g. by exchanging its optical elements, such as the filters set 190 and/or 192, or the dichroic beam splitter 180 and by selecting the corresponding color conversion matrix 142. The selection of the color conversion matrix 142 may be done automatically depending on the current set-up of the color separation assembly 176 or manually by a user.

The digital reflectance color output image 160 may be displayed on a display 132, which is integral with the medical observation device 100. For example, the display 132 may be integrated in an ocular or eyepiece 104 of the medical observation device 100.

The digital reflectance color output image 160 is preferably generated in real-time, i.e. a digital reflectance color output image 160 is generated from a set of digital color input images 130 before the next set is generated by the at least two cameras 110, 111, 111a.

The medical observation device 100 may comprise a direct optical path 134 from the object 106 through the objective 174 to the eyepiece 104. In such a case, the display may be a translucent display 132 located in the direct optical path 134 or the display may be projected into the direct optical path 134. A beam splitter 136 may be provided to split the light between the optical eyepiece 104 and the digital imaging system 102. In one embodiment, up to 80 % of the light may be directed to the eyepiece 104.

Alternatively, the medical observation device 100 may not have a direct optical path 134 but only display images from the integral display 132. As a further alternative, the medical observation device may not have any display at all.

The medical observation device 100 may comprise an output interface 172 to which one or more (external) displays 182 may be connected. For this, the output interface 172 may comprise standardized connectors and data transmission protocols, such as USB, HDMI, DVI, DisplayPort, Bluetooth and/or others. An external display may be a monitor, 3D goggles, oculars and the like. Any combination of external displays may be connected to output interface 172.

The computer 186 or the data processing device 170 is connected to the digital imaging system 102 using one or more data transmission lines 196. A data transmission line may be wired or wireless, or partly wired and partly wireless. The computer 186 and/or the data processing device 170 may not be bodily integrated in the medical observation device 100 but be physically located remote from the digital imaging system 102. For this, the digital imaging system 102 and the computer 186 and/or the data processing device 170 may be connected to a network, such as a LAN, a WLAN or a WAN, to which also at least one display 182 is connected.

According to a modification, the medical observation device 100 may be stereoscopic but comprise only two cameras, one for each stereoscopic channel. In one stereoscopic channel, the fluorescence-light color camera 111 is used and configured to record selectively white-light reflectance, whereas in the other stereoscopic channel, the white-light color camera 110 is used. Such an arrangement provides a stereoscopic white-light color input image if no fluorescence is used and a monoscopic white-light color input image and a monoscopic fluorescence-light color input image if fluorescence is used. The description above and below applies equally to this configuration.

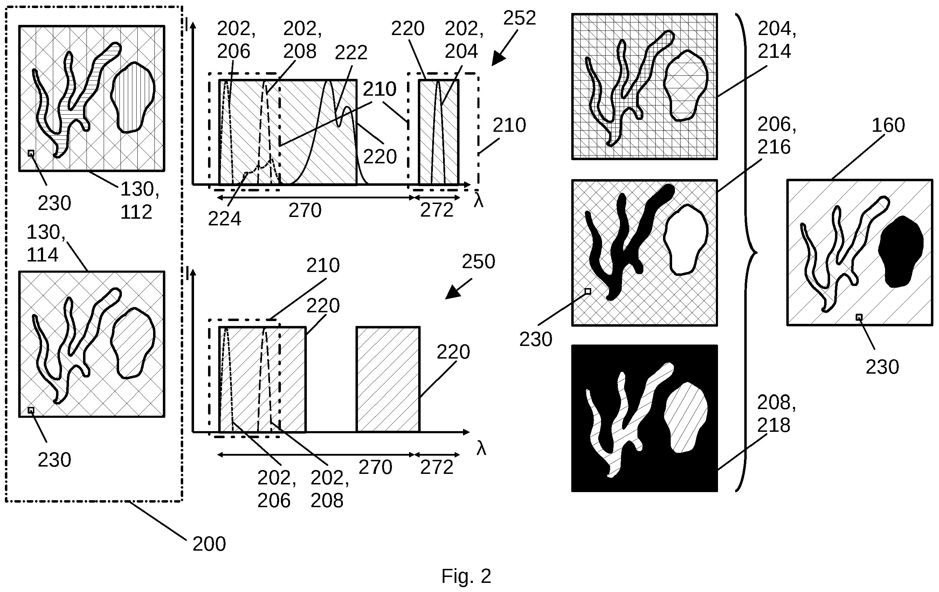

In Fig. 2, a schematic representation is shown how the digital reflectance color output image 160 may be generated from the reflectance signals 202, in particular the supplementary reflectance signal 204, the fluorescence-excitation reflectance signal 206 and the optional second supplementary reflectance signal 208. The reflectance signals 202 may be part of a set 210 of reflectance signals 202. In the embodiment shown in Fig. 2, the reflectance signals 202 are contained in two digital color input images 130, for example a digital fluorescence-light color input image 112 and a digital white-light color input image 114. The digital color input images 130 are part of an input image set 200 that may further comprise a second digital fluorescence-light color input image which may e.g., be recorded by the second digital fluorescence-light color camera 111a of Fig. 1. For simplicity's sake, the second digital fluorescence-light color input image is not shown. In a variant, the second digital fluorescence-light color input image may be used for example instead of the digital white-light color input image 114.

In Fig. 2, an example of a spectrum 252 of the digital fluorescence-light color input image 112 is given. The spectrum 252 may be representative for the spectrum of the entire digital fluorescence-light color input image 112 or may be a sample spectrum at any one pixel 230 of the digital fluorescence-light color input image 112.

The spectrum 252 is composed of several preferably discrete components, which are termed signals and which can be separated from each other and/or extracted individually from the spectrum 252 by e.g. spectral unmixing. The various signals may e.g. be generated by illuminating the object 106 simultaneously with preferably narrow-band wavelength bands which results in a reflection of these wavelength bands. These signals are termed reflectance signals, as they are representative of the reflectance of the object in these wavelength bands. Part of the various signals may be fluorescence-emission signals that represent the fluorescence, in particular in a part of the fluorescence-emission spectrum, of at least one fluorophore.

For example, the digital fluorescence-light color input image 112 contains the fluorescence-emission of at least one fluorophore 116, 118. One such fluorescence-emission may e.g. be represented in the fluorescence-emission signal 222 of one such fluorophore, e.g., 5-ALA. The digital fluorescence-light color input image 112 may also contain the fluorescence-emission of another fluorophore, which is indicated as a second fluorescence-emission signal 224. The second fluorescence-emission signal may be e.g., the autofluorescence of a (second) fluorophore which is naturally contained in the object 106 or of another artificially added (second) fluorophore such as ICG. The second fluorescence-emission signal 224 may alternatively be contained in the second digital fluorescence-light color input image.

In addition to the at least one fluorescence-emission signal 222, 224, the digital fluorescence-light color input image 112 may contain an fluorescence-excitation reflectance signal 206. The excitation fluorescence-reflectance signal 206 is generated by light reflected off the object 106 and located in the fluorescence-excitation spectrum of the fluorophore generating the fluorescence-emission signal 222. For example, the fluorescence-excitation reflectance signal 206 may be the reflected light in the fluorescence excitation spectrum of 5-ALA.

The digital fluorescence-light color input image 112 may contain a supplementary reflectance signal 204 may be generated by illuminating the object preferably in a wavelength band with light having wavelengths that are spaced apart from the other reflectance signals 202 and the fluorescence-emission signals 222, 224. The supplementary reflectance signal 204 is preferably entirely located in the NIR and/or consists of wavelengths larger than 700 nm.

Optionally, a second supplementary reflectance signal 208 may be present. The second supplementary reflectance signal 208 is preferably located in a wavelength band, which overlaps the spectrum of the second fluorescence emission signal of the second fluorophore, i.e. a fluorophore not generating the fluorescence-emission signal and/or not having the fluorescence-excitation spectrum in which the wavelengths of the fluorescence-excitation reflectance signal are located.

The second supplementary reflectance signal 208 may be generated by illuminating the object 106 in a narrow frequency band which overlaps the spectrum of the second fluorescence emission signal 224. Thus, instead of recording the, in many cases weak, second fluorescence emission signal, a stronger, second supplementary reflectance signal 208 is recorded in the digital fluorescence-light color input image 112 or the second digital fluorescence-light color input image at the same location in the spectrum to generate a reflectance signal using already-existing passbands in the optical filters.

To record the signals 206, 222, 204 and optionally 208, the recorded light may have undergone optical filtering e.g. by the color separation assembly 176 and/or the optical filter assembly 187, which may include pass bands 220 in which the signals 202, 222, 224 are contained. These passbands have already been described in the context of Fig. 1 with respect to the optical observation filter assembly 187, the white-light filter set and the fluorescence filter set.

Thus, the digital fluorescence-light color input image 112 may contain at least two reflectance signals 204, 206, 208 and at least one fluorescence-emission signal 222.

The digital white-light color input image 114 may alternatively contain any one of the reflectance signals 204, 206, 208.

As can be seen in Fig. 2, the spectra 250, 252 of the digital color input images 130 preferably complement each other to cover completely the entire visible light range 270. The supplementary reflectance signal 204 may be located in the NIR range 272.

As can be further seen in Fig. 2, each of the reflectance signals 202 represents a separate, different digital reflectance image of the object 106 in a discrete wavelength band, in which preferably the object was illuminated. For example, the supplementary reflectance signal 204, when extracted from the digital fluorescence-light color input image 112 may result in a digital reflectance image 214 which represents what would be seen if the object 204 is illuminated by light in this part of the spectrum, e.g., by light passing through a respective optical pass band 220 and/or generated in this wavelength band.

In the same manner, the fluorescence-excitation reflectance signal 206 represents a reflectance image 216 if extracted from the digital fluorescence-light color input image 112.

Finally, the second supplementary reflectance signal 208 represents a digital reflectance image 218.

The digital reflectance images 214, 216, 218 are preferably color images, i.e., are represented in a color space and contain more than one color space coordinate or color band.

The digital reflectance color output image 160 is then generated from the two or more reflectance signals 202 extracted from the digital color input images 130 e.g., by combining the reflectance images 214, 216 and optionally 218.

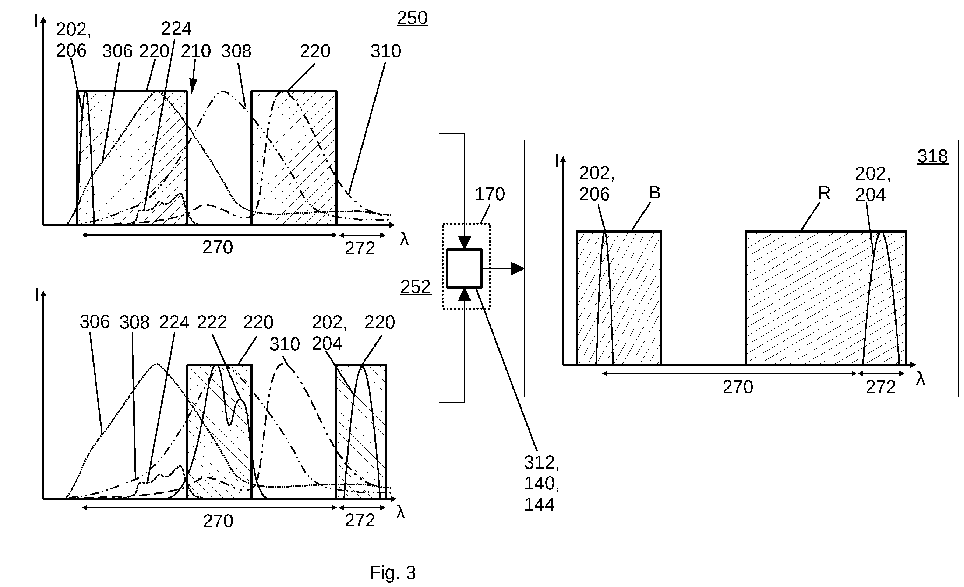

In Fig. 3, it is schematically shown how a digital reflectance color output image 160, being represented by a spectrum 318 is obtained from two digital input images 130 having spectra 250, 252, respectively. In Fig. 3, sample sensitivity curves 306, 308, 310 of sensors of a camera 110, 111, 111a are indicated. Each of the sensitivity curves 306, 308, 310 represents a color space coordinate. For example, the sensitivity curve 306 corresponds to the sensor curve of a first sensor such as the blue sensor in RGB color space. The sensitivity curve 308 represents an example of the sensitivity of a second sensor recording a second color space coordinate, such as the green color space coordinate in RGB color space. Finally, sensitivity 310 is an example of the sensitivity curve of a third sensor recording a third color space coordinate such as the red color space coordinate. Even in the NIR range 272, not only the R sensor, but also the B and the G sensor may still record intensities I. Thus, even light in the NIR range may comprise non-negligible color space coordinate values in the non-red color bands.

In the example of Fig. 3, the spectrum 250, e.g. the spectrum of a digital white-light color input image 112, contains the fluorescence-excitation reflectance signal 206, and the spectrum 252, for example the spectrum of the digital fluorescence-light color input image, contains only the supplementary reflectance signal 204.

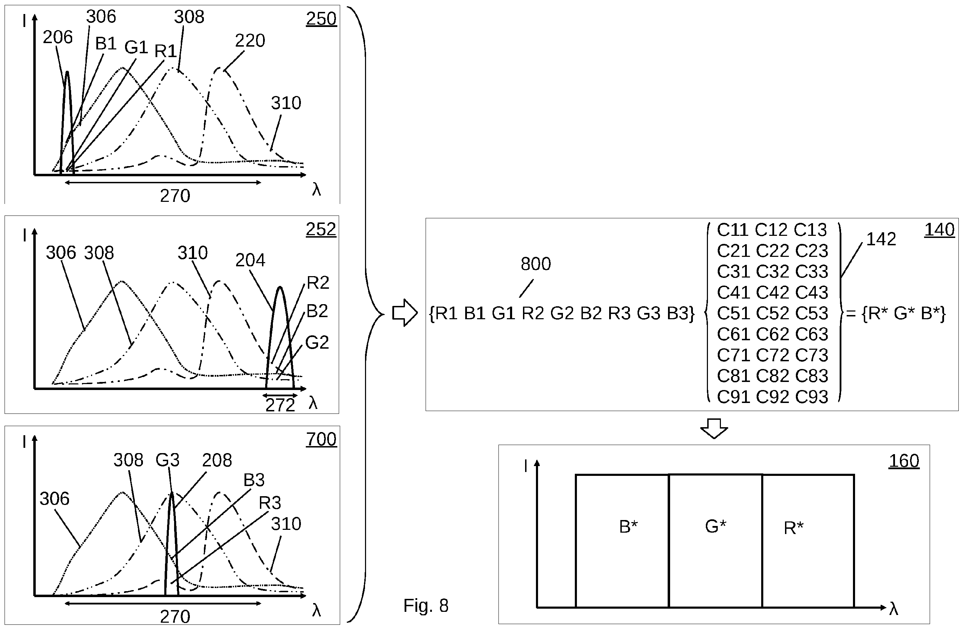

The data processing device 170 (Fig. 1) is configured to extract the reflectance signal 202 from the color input images 130 as indicated by the reference numeral 312. The extraction 312 may comprise applying a linear transformation 140 and/or spectral unmixing 144 to either each of the digital color input images 130 individually or jointly. Two or more digital color input images 130 may, for example, be jointly processed by simply joining their color space coordinates at each pixel 230. Thus, for joint processing, each pixel 230 of the joint image contains six color space coordinates if an RGB color space is used, namely, the three color space coordinates from the first digital color input image 130 and the three color space coordinates from the second digital color input image 130. The joining of color space coordinates is explained further below with reference to Fig. 8.

The digital reflectance color output image 160 here only contains the two reflectance signals 204, 206. In the example given, the fluorescence-excitation reflectance signal 206 is located mainly in the blue and thus represents the blue color space coordinate B in the spectrum 318 of the digital reflectance color output image 160. The supplementary reflectance signal 204, if e.g. located in the NIR range 272, may represent the red color space coordinate R in the spectrum 318. Using linear transformation 140, color conversion may be applied to give the colors represented in the reflectance signal 206, 204 in spectrum 318 a more natural appearance. This is indicated by the broadened color bands B, R in spectrum 318 which extend over the spectral bandwidth of the original signals 204, 206.

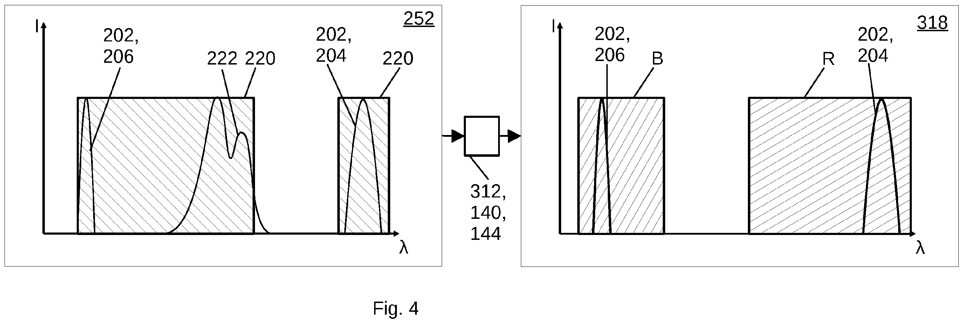

Fig. 4 shows a simplified case, where the reflectance signals 204, 206 are contained in a single digital color input image 130, represented by the spectrum 252. In one embodiment, this single digital color input image 130 may be the digital fluorescence-light color input image 112.

The only difference to the example given in Fig. 3 is that in Fig. 4, the reflectance signals 204, 206 must be extracted from a single image, whereas in Fig. 2 these two reflectance signals are extracted from two different digital color input images 130. Once the reflectance signals 204, 206 are extracted, the digital reflectance color output image 160 is generated as in Fig. 3.

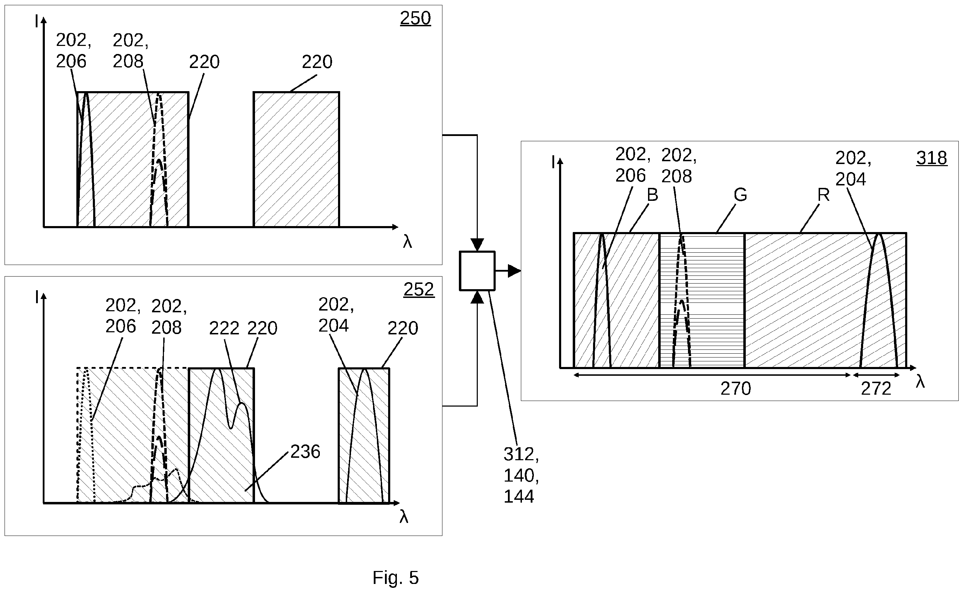

In the example of Fig. 5, it is shown that each of the reflectance signals 204, 206 and, if present, 208, may each be located in the color band of a different primary color of the color space. Thus, the spectrum of each of the reflectance signals 204, 206 and optionally 208 is located at or close to the maximum of a different sensitivity curve 306, 308, 310. Thus, each of the different reflectance signals 202 is, by itself, already representative for a primary color in the digital reflectance color output image 160. Still, it is preferred to not only consider the color space coordinate having the highest intensity value in each of the reflectance signals 202, but also the color space coordinates that have smaller intensity values in order to improve the accurateness of the digital reflectance color output image 160.

In Fig. 5, for example, the supplementary reflectance signal 204 is located in the red color band R and may be used as a representation of the red color space coordinate R in the digital reflectance color output image 160, as shown in spectrum 318. The fluorescence-excitation reflectance signal 206 is located in the blue color band and thus may be a representation of the reflectance of the object in the blue color space coordinate B. The second supplementary reflectance signal 208, if present, may be located in the green color band and thus be representative for the green color space coordinate G in the digital reflectance color output image 160.

As can be seen from the sensitivity curves 306, 308, 310, although each of the reflectance signals 206, 208, 204 is located in a different color band R, G, B, there is still a sensitivity of the other sensitivity curves in each of the color bands. Thus, although the supplementary reflectance signal 204 is in the red color range R, the representation of the color of the supplementary reflectance signal 204 in RGB color space may also contain green and blue color components. This applies mutatis mutandis to the other two reflectance signals 206, 208.

In order to improve color accuracy, it is therefore preferred to apply a linear transformation, e.g. in form of a multiplication by a color conversion matrix 144 which may have been obtained by a color calibration process. The linear transformation 140 maps the colors in the reflectance signals 206, 208, 204 to more naturally-looking colors. Thus, the more narrow-bands spectra of the reflectance signals 206, 208, 204 may be broadened to cover more colors, as shown in the broadened R, G, B spectra in spectrum 318. The application of a color conversion matrix is explained further below with reference to Fig. 8.

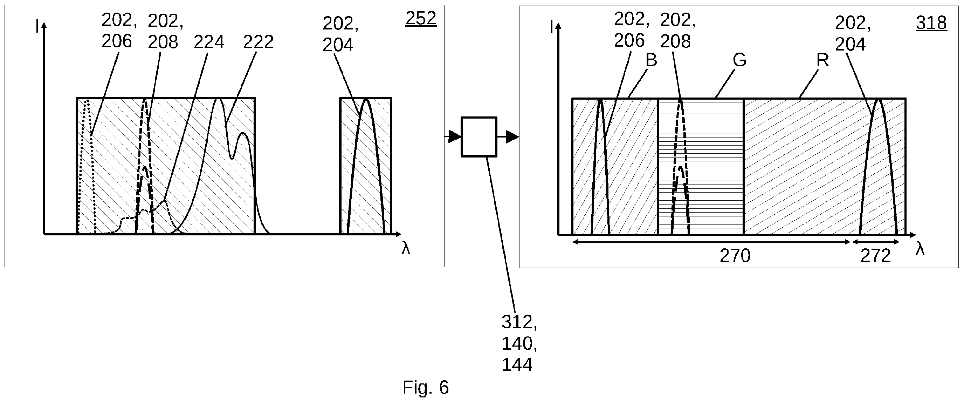

Fig. 6 is an example where three reflectance signals 206, 208 and 204 are contained in a single digital color input image 130. Thus, all three reflectance signals 206, 208, 204 need to be extracted from a single digital color input image 130 and separated from one another prior to generating the digital reflectance color output image 160. In particular, the reflectance signals need to be separated from any fluorescence-emission signal, as the fluorescence-emission signal does not contain any information about the reflectance of the object..

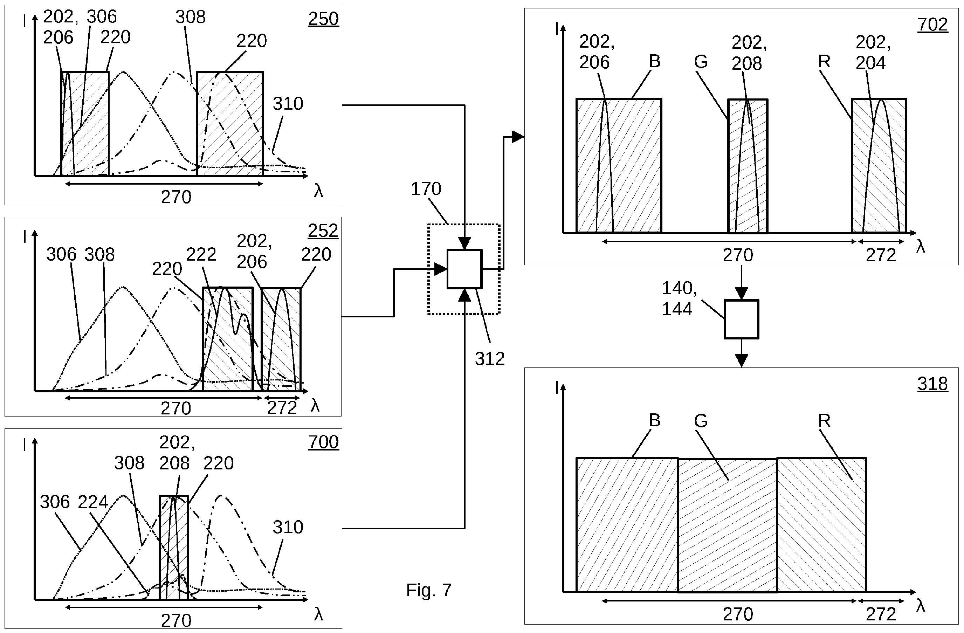

Fig. 7 is an example where three digital input images 130 as represented by the spectra 250, 252 and 700 are used to generate a digital reflectance color output image 130 as represented by spectrum 318.

The third digital color input image 130, represented by spectrum 700, may for example be a second digital fluorescence-light image, which preferably is also a color image. In the example indicated in Fig. 7, each of the spectra 250, 252, 700 contains a different reflectance signal 202. The data processing device 170 uses an extraction 312, such as spectral unmixing, in order to extract the reflectance signals 202 which then may be combined into an intermediate color image of which the spectrum 702 is shown. The intermediate color image does not need to be present in the data processing device 170 as an image, but may also be composed of different data sets which are processed together as an image and thus form a "virtual" image, i.e. a set of separate data that are collectively processed as a single image. In order to adjust the colors of spectrum 702, a linear transformation 140, e.g. by application of a color conversion matrix 144, is applied so that the final digital reflectance color output image 160 is obtained.

In a further process, the data processing device 170 may be configured to also extract the at least one fluorescence-emission signal 222, 224 and mix it with the digital reflectance color output image 130, e.g. using a different pseudo-color for each of the fluorescence-emission signals 222, 224.

Fig. 8 provides an example how a linear transformation 140 using a color conversion matrix 142 applied to the extracted reflectance signals 206, 204 and, optionally, 208 may result in a digital reflectance color output image 160.

At a pixel 230 of a digital color input image 130, the reflectance signal 206 is represented, e.g. in an RGB color space, by the set {R1 G1 B1} of color space coordinates.

At the same pixel, or a corresponding pixel of another digital color input image 130, the reflectance signal 204 is represented by the set {R2 G2 B2} of color space coordinates.

Again at the same pixel, in a corresponding pixel of yet another digital color input image 130, the reflectance signal 208 is represented by the set {R3 G3 B3} of color space coordinates.

After extraction, a union set 800, {R1 G1 B1 R2 G2 B2 R3 G3 B3}, is formed from the three sets of color space coordinates of the reference signals from which the digital reflectance color output image 160 is generated. The union set is then multiplied by the color conversion matrix 142 to result in the color space coordinates {R* G* B*} at the corresponding pixel in the digital reflectance color output image 160. The color conversion matrix has elements C11, C12, C13, C21, ..., C93.

The color conversion matrix 142 has two dimensions. In one of the two dimensions, the number of elements in the color conversion matrix corresponds to the number of color space coordinates in the digital reflectance color output image 160. If the digital reflectance color output image 160 is represented in an RGB color space, the number of elements in this direction is three. Other color spaces may have more color space coordinates and thus require more elements.

In the other one of the two dimension, the number of elements in the color conversion matrix corresponds to the total number of color space coordinates in all the reflectance signals that are used for generating the digital reflectance color output image 160. In the example shown in Fig. 8, this number is 9, as each of the reflectance signals is represented by three color space coordinates. Thus, the matrix has dimension 3x9, or, equivalently, 9x3, depending on the way the matrix multiplication is carried out. If e.g. the reflectance signal 208 is omitted, the union set 800 will be reduced to {R1 G1 B1 R2 G2 B2} and the dimension of the color conversion matrix 142 will be 3x6 or 6x3.

Instead of using extracted reflectance signals, the original digital color input image 130 may be used in which one or more reflectance signals 202 are contained together with other signals, such as fluorescence-emission signals 222, 224. In this case a different color conversion matrix may be used. Otherwise the process stays the same.

The color conversion matrix 142 may be determined by color calibration. The color conversion matrix 142 maps the union set to a more natural-looking output color in the digital reflectance color output image 160.

The data processing device 170 may comprise a plurality of different color conversion matrices 142. In such a case, it may depend on union set 800, which of the plurality of different color conversion matrices is used at a pixel.

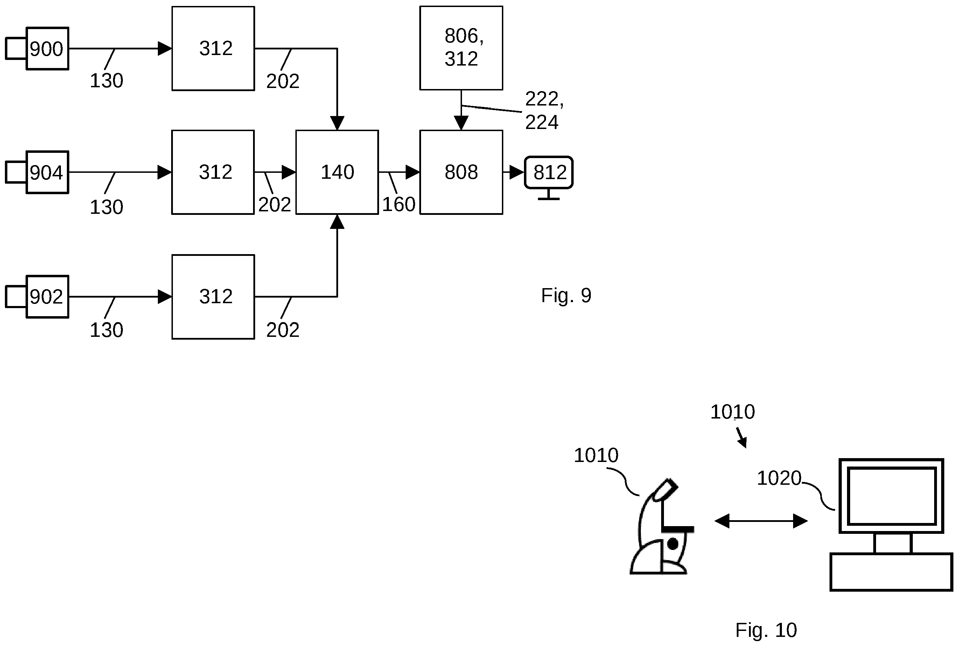

Fig. 9 presents a schematic overview of the different process steps that are carried out to obtain a digital reflectance color output image 130.

At step 900, a first digital color input image 300, e.g., the digital fluorescence-light color input image 112 is recorded.

At step 902, a second digital color input image 130 is recorded e.g., the digital white-light color input image 114.

At optional step 904, a third digital color input image 130 may be recorded, e.g., a second digital fluorescence-light color input image.

At an optional step 312, the reflectance signals 202 may be extracted from the respective first, second and third digital color input image 130. The simplest form of extraction is to extract a different color space coordinate for each of the different reflectance signals 202. However, a more accurate method of extraction is preferred, such as applying spectral unmixing 312 and/or a linear transformation 140. Instead of or in addition to the extraction, a linear transformation 140 may be used. The linear transformation may be applied to the first, second and third digital color input image 130 simultaneously or individually, directly resulting in the digital reflectance color output image 160. As an alternative, if an extraction step, such as spectral unmixing 312 is used, the linear transformation 140 may be applied individually or jointly to the extracted reflectance signals 202.

In a step 906, one or more fluorescence-emission signals 222, 224 may be extracted from the digital color input images 130.

In step 906, the one or more fluorescence-emission signals 222, 224 may be converted into pseudo color and mixed with the digital reflectance color output image 160 to obtain a combined image which is then displayed in step 812 e.g., on a display 182 (Fig. 1).

The steps 140, 806 and 808 may also be combined in a single step using a linear transformation, such as matrix multiplication.

Der Begriff "und/oder" umfasst alle Kombinationen von einem oder mehreren der zugehörigen aufgeführten Elemente und kann mit "/" abgekürzt werden. Obwohl einige Aspekte im Rahmen einer Vorrichtung beschrieben wurden, ist es klar, dass diese Aspekte auch eine Beschreibung des entsprechenden Verfahrens darstellen, wobei ein Block oder eine Vorrichtung einem Verfahrensschritt oder einer Funktion eines Verfahrensschritts entspricht. Analog dazu stellen Aspekte, die im Rahmen eines Verfahrensschritts beschrieben werden, auch eine Beschreibung eines entsprechenden Blocks oder Elements oder einer Eigenschaft einer entsprechenden Vorrichtung dar.

Some embodiments relate to a microscope comprising a system as described in connection with one or more of the Figs. 1 to 9. Alternatively, a microscope may be part of or connected to a system as described in connection with one or more of the Figs. 1 to 9. Fig. 10 shows a schematic illustration of a system 1000 configured to perform a method described herein. The system 1000 comprises a microscope 1010 and a computer system 1020. The microscope 1010 is configured to take images and is connected to the computer system 1020. The computer system 1020 is configured to execute at least a part of a method described herein. The computer system 1020 may be configured to execute a machine learning algorithm. The computer system 1020 and microscope 1010 may be separate entities but can also be integrated together in one common housing. The computer system 1020 may be part of a central processing system of the microscope 1010 and/or the computer system 1020 may be part of a subcomponent of the microscope 1010, such as a sensor, an actor, a camera or an illumination unit, etc. of the microscope 1010.

The computer system 1020 may be a local computer device (e.g. personal computer, laptop, tablet computer or mobile phone) with one or more processors and one or more storage devices or may be a distributed computer system (e.g. a cloud computing system with one or more processors and one or more storage devices distributed at various locations, for example, at a local client and/or one or more remote server farms and/or data centers). The computer system 1020 may comprise any circuit or combination of circuits. In one embodiment, the computer system 1020 may include one or more processors, which can be of any type. As used herein, processor may mean any type of computational circuit, such as but not limited to a microprocessor, a microcontroller, a complex instruction set computing (CISC) microprocessor, a reduced instruction set computing (RISC) microprocessor, a very long instruction word (VLIW) microprocessor, a graphics processor, a digital signal processor (DSP), multiple core processor, a field programmable gate array (FPGA), for example, of a microscope or a microscope component (e.g. camera) or any other type of processor or processing circuit. Other types of circuits that may be included in the computer system *X20* may be a custom circuit, an application-specific integrated circuit (ASIC), or the like, such as, for example, one or more circuits (such as a communication circuit) for use in wireless devices like mobile telephones, tablet computers, laptop computers, two-way radios, and similar electronic systems. The computer system 1020 may include one or more storage devices, which may include one or more memory elements suitable to the particular application, such as a main memory in the form of random access memory (RAM), one or more hard drives, and/or one or more drives that handle removable media such as compact disks (CD), flash memory cards, digital video disk (DVD), and the like. The computer system *X20* may also include a display device, one or more speakers, and a keyboard and/or controller, which can include a mouse, trackball, touch screen, voice-recognition device, or any other device that permits a system user to input information into and receive information from the computer system 10200.

Some or all of the method steps may be executed by (or using) a hardware apparatus, like for example, a processor, a microprocessor, a programmable computer or an electronic circuit. In some embodiments, some one or more of the most important method steps may be executed by such an apparatus.

Depending on certain implementation requirements, embodiments of the invention can be implemented in hardware or in software. The implementation can be performed using a nontransitory storage medium such as a digital storage medium, for example a floppy disc, a DVD, a Blu-Ray, a CD, a ROM, a PROM, and EPROM, an EEPROM or a FLASH memory, having electronically readable control signals stored thereon, which cooperate (or are capable of cooperating) with a programmable computer system such that the respective method is performed. Therefore, the digital storage medium may be computer readable.

Some embodiments according to the invention comprise a data carrier having electronically readable control signals, which are capable of cooperating with a programmable computer system, such that one of the methods described herein is performed.

Generally, embodiments of the present invention can be implemented as a computer program product with a program code, the program code being operative for performing one of the methods when the computer program product runs on a computer. The program code may, for example, be stored on a machine readable carrier.

Other embodiments comprise the computer program for performing one of the methods described herein, stored on a machine readable carrier.

In other words, an embodiment of the present invention is, therefore, a computer program having a program code for performing one of the methods described herein, when the computer program runs on a computer.

A further embodiment of the present invention is, therefore, a storage medium (or a data carrier, or a computer-readable medium) comprising, stored thereon, the computer program for performing one of the methods described herein when it is performed by a processor. The data carrier, the digital storage medium or the recorded medium are typically tangible and/or nontransitionary. A further embodiment of the present invention is an apparatus as described herein comprising a processor and the storage medium.

A further embodiment of the invention is, therefore, a data stream or a sequence of signals representing the computer program for performing one of the methods described herein. The data stream or the sequence of signals for example may be configured to be transferred via a data communication connection, for example, via the internet.

A further embodiment comprises a processing means, for example, a computer or a programmable logic device, configured to, or adapted to, perform one of the methods described herein.

A further embodiment comprises a computer having installed thereon the computer program for performing one of the methods described herein.

A further embodiment according to the invention comprises an apparatus or a system configured to transfer (for example, electronically or optically) a computer program for performing one of the methods described herein to a receiver. The receiver for example may be a computer, a mobile device, a memory device or the like. The apparatus or system for example may comprise a file server for transferring the computer program to the receiver.

In some embodiments, a programmable logic device (for example, a field programmable gate array) may be used to perform some or all of the functionalities of the methods described herein. In some embodiments, a field programmable gate array may cooperate with a microprocessor in order to perform one of the methods described herein. Generally, the methods are preferably performed by any hardware apparatus.

### Reference Numerals

- 100: medical observation device
- 101L: stereoscopic subassembly for the left channel
- 101R: stereoscopic subassembly for the right channel
- 102: digital imaging system
- 104: eyepiece
- 106: object
- 107: biological tissue
- 108: digital color camera
- 110: digital white-light color camera
- 111: digital fluorescence-light color camera
- 111a: second digital fluorescence-light color camera (capturing different spectrum than 111)
- 112: digital fluorescence-light color input image
- 114: digital white-light color input image
- 116: fluorophore
- 118: second fluorophore
- 128: preprocessing routine
- 130: digital color input image
- 132: internal display
- 134: direct optical path
- 136: beam splitter
- 140: linear transformation
- 142: color conversion matrix
- 144: spectral unmixing
- 160: digital reflectance color output image
- 170: data processing device
- 172: output interface
- 174: objective
- 176: color-separation assembly
- 178: illumination assembly
- 179: illumination filter
- 180: illumination beam splitter
- 182: display
- 184: field of view
- 186: computer
- 187: observation filter assembly
- 188: white-light filter set
- 190: fluorescence filter set
- 192: dichroic beam splitter
- 194: memory
- 196: data transmission line
- 199: light source
- 200: input image set
- 202: reflectance signal
- 204: supplementary reflectance signal
- 206: fluorescence-excitation reflectance signal
- 208: second supplementary reflectance signal
- 210: set of reflectance signals
- 214: digital image of object as represented in the supplementary reflectance signal
- 216: digital image of object as represented in the fluorescence-excitation reflectance signal
- 218: digital image of object as represented in the second supplementary reflectance signal
- 220: passband
- 222: fluorescence-emission signal
- 224: second fluorescence-emission signal
- 230: pixel
- 250: spectrum of digital white-light color input image
- 252: spectrum of digital fluorescence-light color input image
- 270: visible light
- 272: NIR range
- 306: sensitivity curve of a first sensor recording a first color space coordinate (B)
- 308: sensitivity curve of a second sensor recording a second color space coordinate (G)
- 310: sensitivity curve of a third sensor recording a third color space coordinate (R)
- 312: extraction of reflectance signal
- 318: spectrum of digital reflectance color output image
- 700: spectrum of second digital fluorescence-light input image
- 702: spectra of the extracted reflectance signals
- 800: union set
- 900: recording a first digital color input image
- 902: recording a second digital color input image
- 904: recording a third digital color input image
- 906: extracting at least one fluorescence emission signal
- 908: mixing the fluorescence emission signal with the digital reflectance color output image
- B, B1, B2, B3, B*: color space coordinate
- G, G1, G2, G3, G*: color space coordinate
- I: intensity
- R, R1, R2, R3, R*: color space coordinate
- RGB: color space
- λ: wavelength

## Claims

1. Data processing device (170) for a medical observation device (100), such as a microscope or an endoscope, for observing an object (106) which contains a fluorophore (116, 118),
wherein the data processing device (170) is configured:
to access an input image set (200), the input image set (200) comprising at least one digital color input image (130) representing an image of the object (106), the input image set (200) containing in the at least one digital color input image (130)
- a fluorescence-emission signal (222) which is representative of emitted fluorescence of the fluorophore (116, 118) in the object (106), and
- a set of reflectance signals (202), each reflectance signal (202) representing a reflectance image of the object (106) in a different wavelength band, the set of reflectance signals (202) containing
= a fluorescence-excitation reflectance signal (206) which is representative of light reflected off the object (106) at wavelengths in the fluorescence excitation spectrum of the fluorophore (116, 118), and
= a supplementary reflectance signal (204) which is representative of light reflected off the object (106) in a wavelength band which is spaced apart from the wavelengths represented in the fluorescence-excitation reflectance signal (206) and the fluorescence-emission signal (222); and
to generate a digital reflectance color output image (160) of the object (106) from a combination of the fluorescence-excitation reflectance signal (206) and the supplementary reflectance signal (204).

2. Data processing device (170) according to claim 1,
wherein the set of reflectance signals (202) further comprises
- a second supplementary reflectance signal (208) which is representative of light reflected off the object (106) in a second wavelength band which is spaced apart from the wavelengths represented in the fluorescence-excitation reflectance signal (206) and the fluorescence-emission signal (222), and from the wavelength band of the supplementary reflectance signal (204); and wherein the data processing device (170) is configured
- to generate the digital reflectance color output image (160) from a combination of the fluorescence-excitation reflectance signal (206), the second supplementary reflectance signal (208) and the supplementary reflectance signal (204).

3. Data processing device (170) according to claim 1 or 2,
wherein the data processing device (170) is configured to
- extract at least one reflectance signal (202) from at least one digital color input image (130) of the input image set (200) by at least one of spectral unmixing (144), linear transformation (140) and extraction of a color space coordinate prior to generating the digital reflectance color output image (160).

4. Data processing device (170) according to claim 3,
wherein the data processing device (170) is configured to
- apply a linear transformation (140) to at least one reflectance signal extracted by spectral unmixing (144).

5. Data processing device (170) according to claim 3 or 4,
wherein the linear transformation (140) comprises a multiplication of the at least one color input image (130), which contains the at least one reflectance signal (202) that is to be extracted, by a color conversion matrix (142) having two dimensions; and
wherein one of the two dimensions corresponds to the number of color-space coordinates (R, G, B) in the digital reflectance color output image (160) and
the other one of the two dimensions corresponds to the total number of color-space coordinates contained in the digital color input images (130) from which the at least one reflectance signal (202) is to be extracted.

6. Data processing device (170) according to any one of claims 1 to 5,
wherein the input image set (200) comprises a digital fluorescence-light color input image (112) as a digital color input image (160);
wherein the fluorescence-light digital color input image (130) contains the fluorescence-emission signal (222) and the supplementary reflectance signal (204); and
wherein the data processing device (170) is configured to
extract the supplementary reflectance signal (204) from the fluorescence-light digital color input image (130) prior to generating the digital reflectance color image.

7. Data processing device (170) according to claim 6,
wherein the digital fluorescence-light color input image (112) contains the fluorescence-excitation reflectance signal (206); and
wherein the data processing device (170) is configured
to extract the fluorescence-excitation reflectance signal (206) from the fluorescence-light digital color input image (130) prior to generating the digital reflectance color image.

8. Data processing device (170) according to any one of claims claim 1 to 7,
wherein the input image set (200) comprises a digital white-light color input image (114) as a digital color input image (130);
wherein the digital white-light color input image (114) contains the fluorescence-excitation reflectance signal (206); and
wherein the data processing device (170) is configured
to extract the fluorescence-excitation reflectance signal (206) from the digital white-light color input image (114) prior to generating the digital reflectance color output image (160).

9. Data processing device (170) according to any one of claims 1 to 8,
wherein the wavelength band that is represented by the supplementary reflectance signal is contained in the NIR-range (304) of the spectrum.

10. Data processing device (170) according to claim 9,
wherein the data processing device (170) is configured to
- generate the color space coordinate (R) representing red light in the digital reflectance color output image (160) from the color space coordinate (R) representing red light of the supplementary reflectance signal (204).

11. Medical observation device (100), such as a microscope or endoscope, the medical observation device (100) comprising:
- a data processing device (170) according to any one of claims 1 to 10;
- a digital fluorescence-light color camera (111) which is configured to record the fluorescence-emission signal (222) and at least one of the reflectance signals (202).

12. Medical observation device (100) according to claim 11,
wherein the medical observation device (100) is configured to illuminate the object (106) with NIR light in the NIR range (304);
wherein the NIR light reflected off the object (106) is represented by the supplementary reflectance signal (204); and
wherein the data processing device (170) is configured to generate at least the color space coordinate (R) representing red light of the digital reflectance color output image (160) depending on the supplementary reflectance signal (204).

13. Computer-implemented method for a medical observation device (100), such as a microscope or an endoscope, for observing an object (106) which contains a fluorophore (116, 118),
the computer-implemented method comprising the steps of:
accessing an input image set (200), the input image set (200) comprising at least one digital color input image (130) representing an image of the object (106), the input image set (200) containing
- a fluorescence-emission signal (222) which is representative of emitted fluorescence of the fluorophore (116, 118) in the object (106),
- a set of reflectance signals (202), each reflectance signal (202) representing a reflectance image of the object (106) in a different wavelength band, the set containing
= a fluorescence-excitation reflectance signal (206) which is representative of light reflected off the object (106) at wavelengths in the fluorescence excitation spectrum of the fluorophore (116, 118),
= a supplementary reflectance signal (204) which is representative of light reflected off the object (106) in a wavelength band which is spaced apart from the wavelengths represented in the fluorescence-excitation reflectance signal (206) and the fluorescence-emission signal (222), and
generating a digital reflectance color output image (160) of the object (106) from a combination of the fluorescence-excitation reflectance signal (206) and the supplementary reflectance signal (204).
14. Method of observing an object (106) containing at least one flurorophore (116, 118), the method comprising:
- the computer-implemented method of claim 13; and the step of
- recording a digital fluorescence-light color image (112) containing the fluorescence-emission signal (222) and at least one of the reflectance signals (202).
15. A computer program product or computer-readable storage medium, comprising instructions which, when the program is executed by a computer, cause the computer to carry out the method of claim 13.
